(19) Europäisches Patentamt
European Patent Office
Office européen des brevets

(11) Veröffentlichungsnummer: **0 009 693**
**B1**

(12) # EUROPÄISCHE PATENTSCHRIFT

(45) Veröffentlichungstag der Patentschrift: **25.11.81**

(51) Int. Cl.³: **C 07 D 277/28,** A 01 N 43/78

(21) Anmeldenummer: **79103460.6**

(22) Anmeldetag: **17.09.79**

(54) **N-(1,3-Thiazolyl)-alkyl-chloracetanilide, Verfahren zu ihrer Herstellung und ihre Verwendung als Herbicide.**

(30) Priorität: **28.09.78 DE 2842280**

(43) Veröffentlichungstag der Anmeldung: **16.04.80 Patentblatt 80/8**

(45) Bekanntmachung des Hinweises auf die Patenterteilung: **25.11.81 Patentblatt 81/47**

(84) Benannte Vertragsstaaten: **AT BE CH DE FR GB IT NL**

(56) Entgegenhaltungen:
**FR-A-2 217 328**
**FR-A-2 368 476**
**FR-A-2 379 525**

(73) Patentinhaber: **BAYER AG, Zentralbereich Patente, Marken und Lizenzen, D-5090 Leverkusen 1, Bayerwerk (DE)**

(72) Erfinder: **Stetter, Jörg, Dr., Pahlkestrasse 3, D-5600 Wuppertal 1 (DE)**
Erfinder: **Thomas, Rudolf, Dr., Wilkhausstrasse 129, D-5600 Wuppertal 2 (DE)**
Erfinder: **Eue, Ludwig, Dr., Paul-Klee-Strasse 36, D-5090 Leverkusen 1 (DE)**
Erfinder: **Schmidt, Robert Rudolf, Dr., Hahnenweg 5, D-5000 Köln 80 (DE)**

**EP 0 009 693 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# N-(1,3-Thiazolyl)-alkyl-chloracetanilide, Verfahren zu ihrer Herstellung und ihre Verwendung als Herbizide

Die vorliegende Erfindung betrifft neue N-(1,3-Thiazolyl)-alkyl-chloracetanilide, mehrere Verfahren zu ihrer Herstellung sowie ihre Verwendung als Herbizide.

Es ist bereits bekannt geworden, daß man 2,6-Diethyl-N-methoxymethyl-Chloracetanilid zur selektiven Unkrautbekämpfung verwenden kann (vgl. R. Wegler, Chemie der Pflanzenschutz- und Schädlingsbekämpfungsmittel, Band 5, Seite 255, Springer-Verlag [1977]). Diese Verbindung ist jedoch nicht immer ausreichend wirksam und in ihrer Selektivität nicht immer ganz befriedigend.

Weiterhin ist bereits bekannt geworden, daß zahlreiche N-substituierte Halogenacetanilide, in denen das Stickstoffatom des Anilinteiles über eine $CH_2$-Gruppe mit einem gegebenenfalls substituierten Azol über ein N-Atom des Azol-Restes, verbunden ist, gute herbizide Eigenschaften besitzen (vgl. FR-A 2 379 525). Die selektive herbizide Wirksamkeit läßt jedoch in manchen Fällen zu wünschen übrig

Es wurden nun neue N-(1,3-Thiazolyl)alkyl-chloracetanilide der Formel

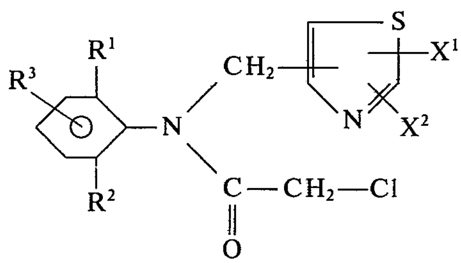

(I)

in welcher

$R^1$ für Alkyl mit 1 bis 4 Kohlenstoffatomen steht,
$R^2$ für Wasserstoff oder Alkyl mit 1 bis 4 Kohlenstoffatomen steht,
$R^3$ für Wasserstoff oder Alkyl mit 1 bis 4 Kohlenstoffatomen steht,
$X^1$ für Wasserstoff, Alkyl mit 1 bis 4 Kohlenstoffatomen oder Phenyl steht,
$X^2$ für Wasserstoff oder Alkyl mit 1 bis 4 Kohlenstoffatomen steht und

der Azolyl-Rest über ein Kohlenstoffatom gebunden ist, gefunden.

Weiterhin wurde gefunden, daß man die N-(1,3-Azolyl)-alkyl-chloracetanilide der Formel (I) erhält, wenn man

a) N-(1,3-Thiazolyl)alkyl-aniline der Formel

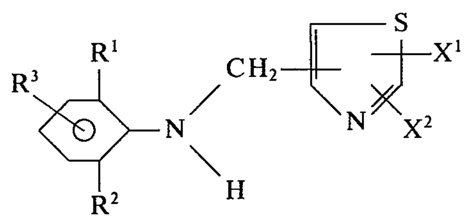

(II)

in welcher

$R^1$, $R^2$, $R^3$, $X^1$ und $X^2$ die oben angegebene Bedeutung haben, und
der Azolyl-Rest über ein Kohlenstoffatom gebunden ist,
mit Chloressigsäurechlorid oder -bromid bzw. -anhydrid der Formeln

$$Cl-CH_2-CO-Cl(Br) \quad (IIIa)$$

bzw.

$$(Cl-CH_2-CO)_2O \quad (IIIb)$$

in Gegenwart eines Verdünnungsmittels und gegebenenfalls in Gegenwart eines Säurebindemittels umsetzt, oder

b) Chloracetanilide der Formel

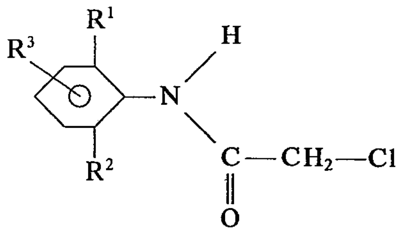

(IV)

in welcher

$R^1$, $R^2$ und $R^3$ die oben angegebene Bedeutung haben,

mit Thiazolyl-alkyl-Derivaten der Formel

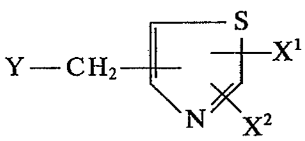

(V)

in welcher

$X^1$ und $X^2$ die oben angegebene Bedeutung haben und
Y für Halogen, den Mesylat- oder Tosylat-Rest steht, und

der Azolyl-Rest über ein Kohlenstoffatom gebunden ist, in Gegenwart eines Säurebinders und gegebenenfalls in Gegenwart eines organischen Lösungsmittels umsetzt.

Die neuen N-(1,3-Thiazolyl)alkyl-chloracetanilide der Formel (I) weisen starke herbizide, insbesondere selektiv-herbizide Eigenschaften auf.

Überraschenderweise zeigen die erfindungsgemäßen N-(1,3-Thiazolyl)alkyl-chloracetanilide bei sehr guter Unkrautwirkung bessere Möglichkeiten zum Einsatz als selektive Unkrautbekämpfungsmittel in wichtigen Kulturpflanzen als das aus dem Stand der Technik bekannte 2,6-Diethyl-N-methoxymethyl-chloracetanilid, welches ein hochaktiver Wirkstoff gleicher Wirkungsart ist.

Ferner übertreffen die erfindungsgemäßen N-(1,3-Thiazolyl)alkyl-chloracetanilide auch konstitutionell ähnliche Stoffe, die in der FR-A 2 379 525 offenbart werden, bezüglich ihrer selektiven herbiziden Wirksamkeit. So lassen sich zum Beispiel die erfindungsgemäßen Stoffe besser zur selektiven Unkrautbekämpfung in Weizen, Raps, Baumwolle und/oder Rüben einsetzen als die vorbekannten Verbindungen

2-Methyl-6-ethyl-N-(2,4,5-imidazol-1-yl-methyl)-chlor-acetanilid,
2,6-Diethyl-N-(imidazol-1-yl-methyl)-chloracetanilid und
2-tert.-Butyl-N-(pyrazol-1-yl-methyl)-chloracetanilid.

Die erfindungsgemäßen N-(1,3-Azolyl)alkyl-chloracet-anilide sind durch die Formel (I) allgemein definiert. Besonders bevorzugt sind diejenigen Stoffe, in denen $R^1$ für Methyl, Ethyl, Isopropyl, sek.-Butyl oder tert.-Butyl steht; $R^2$ für Wasserstoff, Methyl, Ethyl, Isopropyl, sek.-Butyl oder tert.-Butyl steht; $R^3$ für Wasserstoff, Methyl, Ethyl Isopropyl, sek.-Butyl oder tert.-Butyl steht; $X^1$ für Wasserstoff, Methyl, Ethyl, n-Propyl, Isopropyl, n-Butyl, sek.-Butyl, tert.-Butyl oder Phenyl steht und $X^2$ für Wasserstoff, Methyl, Ethyl, n-Propyl, Isopropyl, n-Butyl, sek.-Butyl oder tert.-Butyl steht.

Im einzelnen seien außer den bei den Herstellungsbeispielen genannten Verbindungen die folgenden Verbindungen der allgemeinen Formel (I) genannt:

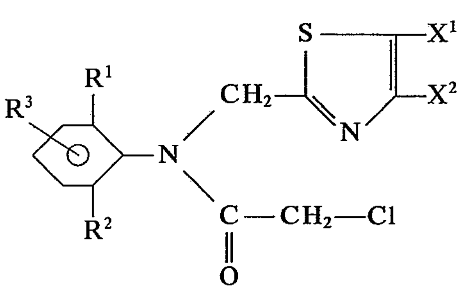

(Ia)

| $R^1$ | $R^2$ | $R^3$ | $X^1$ | $X^2$ |
|---|---|---|---|---|
| $CH_3$ | $CH_3$ | H | H | H |
| $C_2H_5$ | $CH_3$ | H | H | H |
| $C_2H_5$ | $C_2H_5$ | H | H | H |
| $C(CH_3)_3$ | H | H | H | H |
| $CH_3$ | H | 3-$CH_3$ | H | H |
| $CH_3$ | H | 5-$CH_3$ | H | H |
| $CH_3$ | $CH_3$ | 3-$CH_3$ | H | H |
| $CH_3$ | $CH_3$ | H | H | $CH_3$ |
| $C_2H_5$ | $CH_3$ | H | H | $CH_3$ |
| $C_2H_5$ | $C_2H_5$ | H | H | $CH_3$ |
| $C(CH_3)_3$ | H | H | H | $CH_3$ |
| $CH_3$ | H | 3-$CH_3$ | H | $CH_3$ |
| $CH_3$ | H | 5-$CH_3$ | H | $CH_3$ |
| $CH_3$ | $CH_3$ | 3-$CH_3$ | H | $CH_3$ |
| $CH_3$ | $CH_3$ | H | H | $C_2H_5$ |
| $C_2H_5$ | $CH_3$ | H | H | $C_2H_5$ |
| $C_2H_5$ | $C_2H_5$ | H | H | $C_2H_5$ |
| $C(CH_3)_2$ | $CH_3$ | H | H | H |
| $CH_3$ | $CH_3$ | H | H | $C_4H_9$ |
| $C_2H_5$ | $CH_3$ | H | H | $C_4H_9$ |
| $C_2H_5$ | $C_2H_5$ | H | H | $C_4H_9$ |
| $CH_3$ | $CH_3$ | H | H | $C_3H_7$ |
| $C_2H_5$ | $CH_3$ | H | H | $C_3H_7$ |
| $C_2H_5$ | $C_2H_5$ | H | H | $C_3H_7$ |
| $CH_3$ | $CH_3$ | H | $CH_3$ | $CH_3$ |
| $C_2H_5$ | $CH_3$ | H | $CH_3$ | $CH_3$ |
| $C_2H_5$ | $C_2H_5$ | H | $CH_3$ | $CH_3$ |
| $CH_3$ | $CH_3$ | H | $CH_3$ | H |
| $C_2H_5$ | $CH_3$ | H | $CH_3$ | H |
| $C_2H_5$ | $C_2H_5$ | H | $CH_3$ | H |

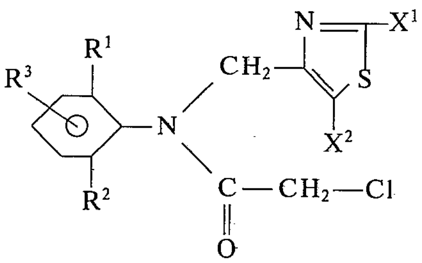

(Ib)

| R¹ | R² | R³ | X¹ | X² |
|---|---|---|---|---|
| $CH_3$ | $CH_3$ | H | H | H |
| $C_2H_5$ | $CH_3$ | H | H | H |
| $C_2H_5$ | $C_2H_5$ | H | H | H |
| $C(CH_3)_3$ | H | H | H | H |
| $CH_3$ | H | 3-$CH_3$ | H | H |
| $CH_3$ | H | 5-$CH_3$ | H | H |
| $CH_3$ | $CH_3$ | 3-$CH_3$ | H | H |
| $C(CH_3)_2$ | $CH_3$ | H | H | H |
| $CH_3$ | $CH_3$ | H | $CH_3$ | H |
| $C_2H_5$ | $CH_3$ | H | $CH_3$ | H |
| $C_2H_5$ | $C_2H_5$ | H | $CH_3$ | H |
| $C(CH_3)_3$ | H | H | $CH_3$ | H |
| $CH_3$ | H | 3-$CH_3$ | $CH_3$ | H |
| $CH_3$ | H | 5-$CH_3$ | $CH_3$ | H |
| $CH_3$ | $CH_3$ | 3-$CH_3$ | $CH_3$ | H |
| $C(CH_3)_2$ | $CH_3$ | H | $CH_3$ | H |
| $CH_3$ | $CH_3$ | H | $C_2H_5$ | H |
| $C_2H_5$ | $CH_3$ | H | $C_2H_5$ | H |
| $C_2H_5$ | $C_2H_5$ | H | $C_2H_5$ | H |
| $C(CH_3)_3$ | H | H | $C_2H_5$ | H |
| $CH_3$ | $CH_3$ | H | $C_3H_7$ | H |
| $C_2H_5$ | $CH_3$ | H | $C_3H_7$ | H |
| $C_2H_5$ | $C_2H_5$ | H | $C_3H_7$ | H |
| $C(CH_3)_3$ | H | H | $C_3H_7$ | H |
| $CH_3$ | $CH_3$ | H | $C_4H_9$ | H |
| $C_2H_5$ | $CH_3$ | H | $C_4H_9$ | H |

Fortsetzung

| $R^1$ | $R^2$ | $R^3$ | $X^1$ | $X^2$ |
|---|---|---|---|---|
| $C_2H_5$ | $C_2H_5$ | H | $C_4H_9$ | H |
| $C(CH_3)_3$ | H | H | $C_4H_9$ | H |
| $CH_3$ | $CH_3$ | H | $C_6H_5$ | H |
| $C_2H_5$ | $CH_3$ | H | $C_6H_5$ | H |
| $C_2H_5$ | $C_2H_5$ | H | $C_6H_5$ | H |
| $C(CH_3)_3$ | H | H | $C_6H_5$ | H |
| $CH_3$ | $CH_3$ | H | $CH_3$ | $CH_3$ |
| $C_2H_5$ | $CH_3$ | H | $CH_3$ | $CH_3$ |
| $C_2H_5$ | $C_2H_5$ | H | $CH_3$ | $CH_3$ |
| $C(CH_3)_3$ | H | H | $CH_3$ | $CH_3$ |

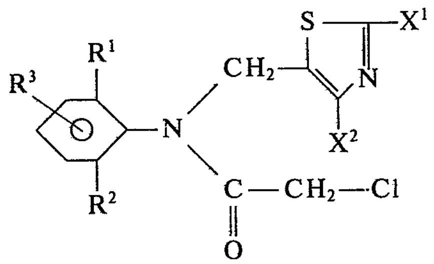

(Ic)

| $R^1$ | $R^2$ | $R^3$ | $X^1$ | $X^2$ |
|---|---|---|---|---|
| $CH_3$ | $CH_3$ | H | H | H |
| $C_2H_5$ | $CH_3$ | H | H | H |
| $C_2H_5$ | $C_2H_5$ | H | H | H |
| $C(CH_3)_3$ | H | H | H | H |
| $CH_3$ | $CH_3$ | H | $CH_3$ | H |
| $C_2H_5$ | $CH_3$ | H | $CH_3$ | H |
| $C_2H_5$ | $C_2H_5$ | H | $CH_3$ | H |
| $CH_3$ | $CH_3$ | H | H | $CH_3$ |
| $C_2H_5$ | $CH_3$ | H | H | $CH_3$ |
| $C_2H_5$ | $C_2H_5$ | H | H | $CH_3$ |

Verwendet man 2,6-Dimethyl-N-(2'-methylthiazol-4'-yl-methyl)anilin und Chloracetylchlorid als Ausgangsstoffe, so kann der durch das folgende Formelschema wiedergegeben werden (Verfahrensvariante a):

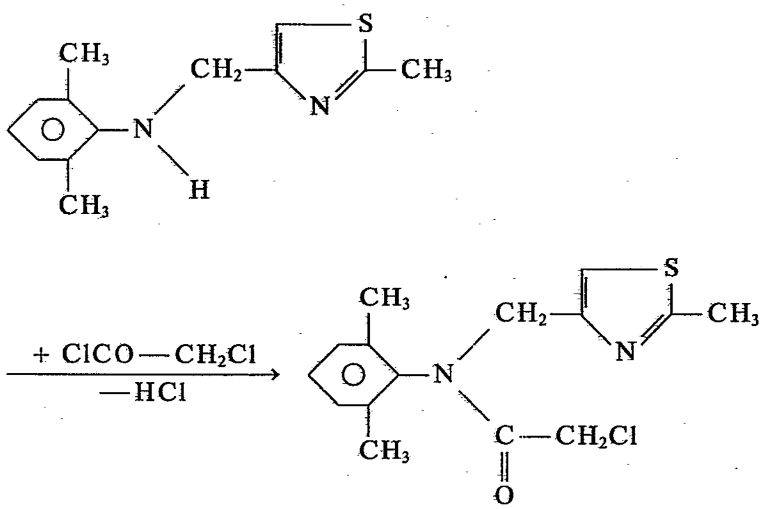


Verwendet man 2-Ethyl-6-methyl-chloracetanilid und 4-Brom-methyl-2-methyl-thiazol als Ausgangsstoffe, so kann der Reaktionsablauf durch das folgende Formelschema wiedergegeben werden (Verfahrensvariante b):

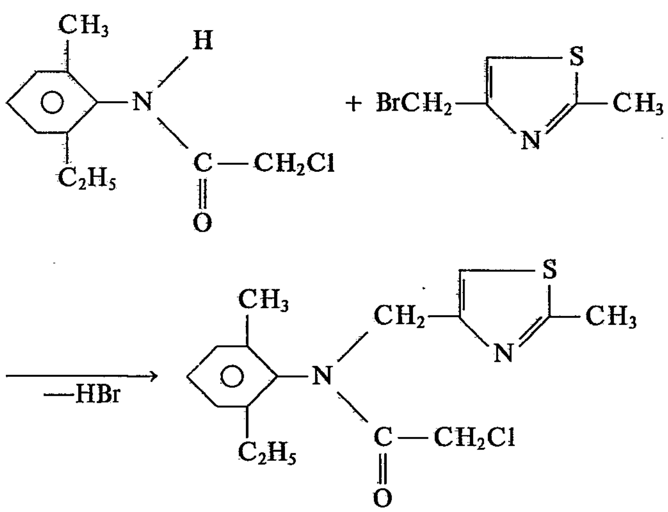


Die bei der Durchführung des erfindungsgemäßen Verfahrens (a) als Ausgangsstoffe benötigten N-(1,3-Azolyl)alkyl-aniline sind durch die Formel (II) allgemein definiert. In dieser Formel stehen $R^1$, $R^2$, $R^3$, $X^1$ und $X^2$ für diejenigen Reste, die bereits im Zusammenhang mit der Beschreibung der erfindungsgemäßen Stoffe der Formel (I) für diese Reste genannt wurden.

Die N-(1,3-Thiazolyl)alkyl-aniline der Formel (II) sind noch nicht bekannt. Man erhält sie, wenn man Aniline der Formel

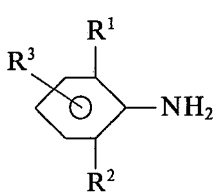
(VI)

in welcher

$R^1$, $R^2$ und $R^3$ die oben angegebene Bedeutung haben,

α) mit Thiazolylalkyl-Derivaten der Formel

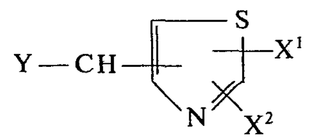

(V)

in welcher

$X^1$, $X^2$ und Y die oben angegebene Bedeutung haben, und der Azolyl-Rest über ein Kohlenstoffatom gebunden ist,

in Gegenwart eines Säurebinders und gegebenenfalls in Gegenwart eines Verdünnungsmittels umsetzt, oder

β) mit Thiazol-aldehyden der Formel

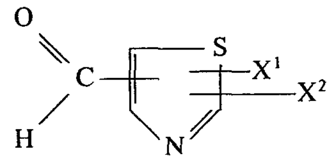

(VII)

in welcher

$X^1$ und $X^2$ die oben angegebene Bedeutung haben, und der Azolyl-Rest über ein Kohlenstoffatom gebunden ist,

in Gegenwart eines inerten organischen Lösungsmittels und gegebenenfalls eines Katalysators umsetzt und die entstehenden Imine der Formel

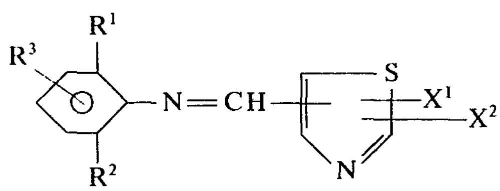

(VIII)

in welcher

$R^1$, $R^2$, $R^3$, $X^1$ und $X^2$ die oben angegebene Bedeutung haben, und der Azolyl-Rest über ein Kohlenstoffatom gebunden ist,

gegebenenfalls in Gegenwart eines polaren Verdünnungsmittels reduziert.

Die bei der Herstellung der N-(1,3-Thiazolyl)alkyl-aniline der Formel (II) als Ausgangsstoffe benötigten Aniline der Formel (VI) sind allgemein bekannte Verbindungen der organischen Chemie. Als Beispiele seien genannt:

Anilin; 2-Methylanilin; 2-Ethylanilin; 2-isopropylanilin; 2-sek.-Butylanilin; 2-tert.-Butylanilin; 2,6-Dimethylanilin; 2,3-Dimethylanilin; 2,5-Dimethylanilin; 2,6-Diethylanilin; 2-Ethyl-6-methyl-anilin; 2,4,6-Trimethylanilin; 2-Ethyl-4,6-dimethylanilin; 2,6-Diethyl-4-methylanilin; 2,6-Diisopropyl-4-methylanilin; 2,3,6-Trimethylanilin.

Die bei der Herstellung der N-(1,3-Thiazolyl)alkylaniline der Formel (II) weiterhin als Ausgangsstoffe benötigten Azol-aldehyde der Formel (VII) sind bekannt (vgl. z. B. J. Am. Chem.Soc. 61, 891 [1939]; Acta Chem. Scand. 20, 2600 (1966) und Helv. Chim. Acta 34, 147 [1959]) bzw. können nach den dort beschriebenen Verfahren erhalten werden.

Bei der Herstellung der N-(1,3-Azolyl)alkyl-aniline der Formel (II) nach dem Verfahren (α) können als

Säurebinder alle üblichen Säureakzeptoren verwendet werden. Vorzugsweise in Betracht kommen Alkalicarbonate wie Kalium- oder Natriumcarbonat.

Als Verdünnungsmittel können bei dem Verfahren (α) alle üblichen inerten organischen Lösungsmittel eingesetzt werden. Vorzugsweise in Betracht kommen Dimethylformamid und Toluol.

Die Reaktionstemperaturen können bei dem Verfahren (α) innerhalb eines größeren Bereiches variiert werden. Im allgemeinen arbeitet man zwischen 0°C und 180°C, vorzugsweise zwischen 20°C und 160°C.

Bei der Umsetzung nach dem Verfahren (α) setzt man die Aniline der Formel (VI) und die Azolylalkyl-Derivate der Formel (V) im allgemeinen in äquimolaren Mengen ein. Es ist jedoch auch möglich, eine der Komponenten, vorzugsweise das Anilin der Formel (VI), in einem Überschuß einzusetzen. — die Aufarbeitung und Isolierung der Reaktionsprodukte erfolgt nach üblichen Methoden (vgl. Herstellungsbeispiele).

Bei der Herstellung der N-(1,3-Thiazolyl)alkylaniline der Formel (II) nach dem Verfahren (β) können in der ersten Stufe als inerte organische Lösungsmittel alle üblichen derartigen Solventien verwendet werden. Vorzugsweise in Betracht kommen aromatische Lösungsmittel, wie Toluol.

Als Katalysatoren können bei der Umsetzung (β) in der ersten Stufe alle für derartige Additionen üblichen Reaktionsbeschleuniger verwendet werden. Vorzugsweise in Frage kommen starke organische Säuren, wie p-Toluolsulfonsäure.

In der zweiten Stufe des Verfahrens (β) können als Lösungsmittel alle inerten polaren organischen Solventien verwendet werden. Vorzugsweise in Betracht kommen Alkohole, wie Methanol.

Als Reduktionsmittel können bei der Durchführung der zweiten Stufe des Verfahrens (β) vorzugsweise komplexe Hydride, wie z. B. Natriumhydrid, verwendet werden.

Die Reaktionstemperaturen können bei der Durchführung des Verfahrens (β) sowohl in der ersten als auch in der zweiten Stufe innerhalb eines größeren Bereiches variiert werden. In der ersten Stufe arbeitet man im allgemeinen bei Temperaturen zwischen 40°C und 140°C, vorzugsweise zwischen 60°C und 120°C. In der zweiten Stufe arbeitet man im allgemeinen bei Temperaturen zwischen −10°C und +100°C, vorzugsweise zwischen 0°C und 80°C.

Bei der Durchführung des Verfahrens (β) setzt man die Aniline der Formel (VI) und die Azol-aldehyde der Formel (VII) im allgemeinen in äquivalenten Mengen ein. Es ist jedoch auch möglich, eine der Komponenten, vorzugsweise das Anilin der Formel (VI), in einem Überschuß einzusetzen. Das in der zweiten Stufe benötigte Reduktionsmittel wird zweckmäßigerweise in einem Überschuß einzusetzen. Das in der zweiten Stufe benötigte Reduktionsmittel wird zweckmäßigerweise in einem Überschuß eingesetzt. — Die Aufarbeitung und Isolierung der Reaktionsprodukte erfolgt jeweils nach üblichen Methoden (vgl. Herstellungsbeispiele).

Die außerdem für das erfindungsgemäße Verfahren (a) als Ausgangsstoffe zu verwendenden Verbindungen Chloressigsäurechlorid und -bromid bzw. -anhydrid sind durch die Formeln (IIIa) und (IIIb) definiert.

Chloressigsäurechlorid und -bromid bzw. -anhydrid der Formeln (IIIa) und (IIIb) sind allgemein bekannte Verbindungen der organischen Chemie.

Die bei der Durchführung des erfindungsgemäßen Verfahrens (b) als Ausgangsstoffe benötigten Chloracetanilide sind durch die Formel (IV) allgemein definiert. In dieser Formel stehen $R^1$, $R^2$ und $R^3$ für diejenigen Reste, die bereits im Zusammenhang mit der Beschreibung der erfindungsgemäßen Stoffe der Formel (I) für diese Reste genannt wurden.

Die Chloracetanilide der Formel (IV) sind allgemein bekannt bzw. können sie in allgemein bekannter Art und Weise erhalten werden, indem man entsprechende Aniline mit einem Chloressigsäurechlorid oder -bromid bzw. -anhydrid der Formeln (IIIa) bzw. (IIIb) in Gegenwart eines inerten organischen Lösungsmittels, wie beispielsweise Toluol oder Dimethylformamid, gegebenenfalls in Gegenwart eines Säurebindemittels, wie z. B. Kaliumcarbonat oder Triethylamin, bei Temperaturen zwischen 0 und 100°C umsetzt (vergleiche auch die Herstellungsbeispiele).

Die außerdem für das erfindungsgemäße Verfahren (b) als Ausgangsstoffe zu verwendenden Azolylalkyl-Derivate sind durch die Formel (V) allgemein definiert. In dieser Formel stehen $X^1$ und $X^2$ für diejenigen Reste, die bereits im Zusammenhang mit der Beschreibung der erfindungsgemäßen Stoffe der Formel (I) genannt wurden. Y steht vorzugsweise für Chlor, Brom, den Mesylat- und Tosylat-Rest.

Die Azolylalkyl-Derivate der Formel (V) sind bekannt (vergleiche u. a. J. Am. Chem. Soc. 56, 470 [1934]; J. Am. Chem. Soc. 73, 2935 [1951] und J. Org. Chem. 25, 1151 [1960]) bzw. können sie in allgemein bekannter Art und Weise erhalten werden, indem man z. B. die entsprechenden Methyl-Derivate nach üblichen Methoden halogeniert.

Als Verdünnungsmittel kommen für die erfindungsgemäße Umsetzung gemäß Verfahrensvariante (a) vorzugsweise inerte organische Lösungsmittel in Frage. Hierzu gehören vorzugsweise Ketone, wie Diethylketone, insbesondere Aceton und Methylethylketon; Nitrile, wie Propionitril, insbesondere Acetonitril; Ether, wie Tetrahydrofuran oder Dioxan; aliphatische und aromatische Kohlenwasserstoffe, wie Petrolether, Benzol, Toluol oder Xylol; halogenierte Kohlenwasserstoffe, wie Methylenchlorid, Tetrachlorkohlenstoff, Chloroform oder Chlorbenzol; und Ester, wie Essigester.

Das erfindungsgemäße Verfahren (a) kann gegebenenfalls in Gegenwart von Säurebindern (Chlorwasserstoff-Akzeptoren) durchgeführt werden. Als solche können alle üblichen säurebindemittel verwendet werden. Hierzu gehören vorzugsweise organische Basen, wie tertiäre Amine, beispielsweise Triethylamin, oder Pyridin, ferner anorganische Basen, wie beispielsweise Alkalihydroxide und Alkalicarbonate.

Die Reaktionstempraturen können bei der Durchführung des erfindungsgemäßen Verfahrens (a) in einem größeren Bereich variiert werden. Im allgemeinen arbeitet man zwischen 0° C und 120° C, vorzugsweise zwischen 20° C und 100° C.

Bei der Durchführung des erfindungsgemäßen Verfahrens () setzt man vorzugsweise auf 1 Mol der Verbindung der Formel (II) 1 bis 1,5 Mol Chloracetylierungsmittel und 1 bis 1,5 Mol Säurebinder ein. Die Isolierung der Verbindungen der Formel (I) erfolgt in üblicher Weise.

Als Verdünnungsmittel kommen für die erfindungsgemäße Umsetzung gemäß Verfahren (b) alle inerten, mit Wasser nicht mischbaren, organischen Lösungsmittel in Frage. Hierzu gehören vorzugsweise Ether, wie Dietylether; aromatische Kohlenwasserstoffe, wie Benzol, Toluol oder Xylol; halogenierte Kohlenwasserstoffe, wie Methylenchlorid, Tetrachlorkohlenstoff, Chloroform oder Chlorbenzol; und Ester, wie Essigester.

Die erfindungsgemäße Umsetzung gemäß Verfahren (b) wird in Gegenwart eines Säurebindemittels durchgeführt. Als solche können alle üblichen Säurebindemittel verwendet werden. Hierzu gehören vorzugsweise anorganische Basen, wie beispielsweise Alkalihydroxide und Alkalicarbonate.

Die Reaktionstemperaturen können bei der Durchführung des erfindungsgemäßen Verfahrens (b) in einem größeren Bereich variiert werden. Im allgemeinen arbeitet man zwischen $-70°$ C und $+100°$ C, vorzugsweise zwischen $-20°$ C und $+80°$ C.

Bei der Durchführung des erfindungsgemäßen Verfahrens (b) setzt man vorzugsweise auf 1 Mol Chloracetanilid der Formel (IV) 1 bis 1,5 Mol Azolyl-alkyl-Derivat der Formel (V) ein. Die Isolierung der Verbindungen der Formel (I) erfolgt in üblicher Weise.

In einer bevorzugten Ausführungsform wird die erfindungsgemäße Umsetzung gemäß Verfahren (b) in einem Zweiphasensystem, wie beispielsweise wäßrige Natron- oder Kalilauge/Toluol oder Methylenchlorid, gegebenenfalls unter Zusatz von 0,1 – 1 Mol eines Phasen-Transfer-Katalysators, wie beispielsweise Ammonium- oder Phosphoniumverbindungen, beispielsweise seien Benzyl-dodecyldimethyl-ammoniumchlorid (Zephirol) und Triethyl-benzyl-ammoniumchlorid genannt, durchgeführt (vgl. auch die Herstellungsbeispiele).

Die erfindungsgemäßen Wirkstoffe beeinflussen das Pflanzenwachstum und können deshalb als Defoliants, Desiccants, Krautabtötungsmittel, Keimhemmungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z. B. bei den folgenden Pflanzen verwendet werden:

Dikotyle Unkräuter der Gattungen:

Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dicotyle Kulturen der Gattungen:

Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cuburbita.

Monokotyle Unkräuter der Gattungen:

Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattung:

Oryzae, Zea, Triticum, Hordeum, Avena, Secala, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen ich in Abhängigkeit von der Konzentration zur Totalunkrautbekämpfung z. B. auf Industrie- und Gleisanlagen und auf Wegen und Plätzen mit und ohne Baumbewuchs. Ebenso können die Verbindungen zur Unkrautbekämpfung in Dauerkulturen z. B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe zeigen insbesondere neben einer sehr guten Wirkung gegen grasartige Unkräuter und gegen Cyperus-Arten auch eine gute herbizide Wirkung bei breitblättrigen

Unkräutern. Ein selektiver Einsatz der erfindungsgemäßen Wirkstoffe ist möglich, vorzugsweise in Getreide, Raps, Baumwolle und Zuckerrüben.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsionskonzentrate, Wirkstoff-imprägnierte Natur- und synthetische Stoffe, Feinverkapselungen in polymeren Stoffen.

Diese Formulierunge werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z. B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z. B. nichtionogene und anionische Emulgatoren, wie Polyoxyäthylen-Fettsäure-Ester, Polyoxyäthylen-Fettalkohol-Äther, z. B. Alkylarylpolyglykol-äther, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe, wie anorganische Pigmente, z. B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molbydän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können als solche oder in ihren Formulierungen auch in Mischung mit bekannten Herbiziden zur Unkrautbekämpfung Verwendung finden, wobei Fertigformulierung oder Tankmischung möglich ist. Auch eine Mischung mit anderen bekannten Wirkstoffen, wie Fungiziden, Insektiziden, Akariziden, Nematiziden, Schutzstoffen gegen Vogelfraß, Wuchsstoffen, Pflanzennährstoffen und Bodenstrukturverbesserungsmitteln ist möglich.

Die Wirksoffe können als solche, in Form ihrer Formulierungen oder der daraus durch weiteres Verdünnen bereiteten Anwendungsformen, wie gebrauchsfertige Lösungen, Suspensionen, Emulsionen, Pulver, Pasten, Granulate angewandt werden. Die Anwendung geschieht in üblicher Weise, z. B. durch Gießen, Spritzen, Sprühen, Streuen.

Die erfindungsgemäßen Wirkstoffe können sowohl vor als auch nach dem Auflaufen der Pflanzen appliziert werden. Die Anwendung wird vorzugsweise vor dem Auflaufen der Pflanzen, also im pre-emergence-Verfahren, vorgenommen. Sie können auch vor der Saat in den Boden eingearbeitet werden.

Die aufgewandte Wirkstoffmenge kann in größeren Bereichen schwanken. Sie hängt im wesentlichen von der Art des gewünschten Effekts ab. Im allgemeinen liegen die Aufwandmengen zwischen 0,1 und 10 kg Wirkstoff pro ha, vorzugsweise zwischen 0,1 und 5 kg/ha.

In dem nachfolgenden Beispiel werden die nachstehend angegebenen Verbindungen als Vergleichssubstanzen eingesetzt:

A =
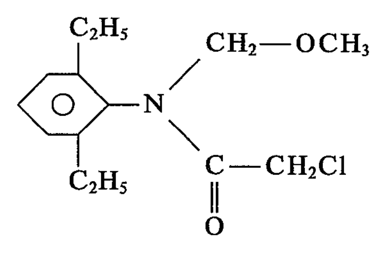

(2,6-Diethyl)-N-methoxymethyl-chloracetanilid)

(B) = 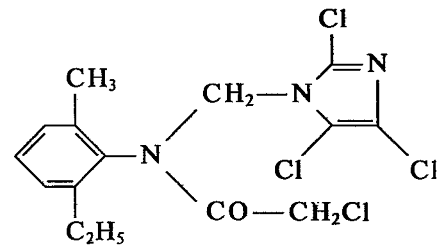

(bekannt aus FR-A 2 379 525)

(C) = 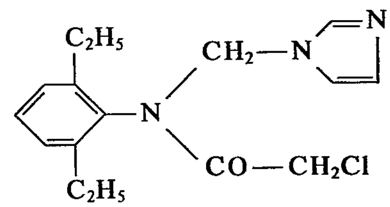

(bekannt aus FR-A 2 379 525)

(D) = 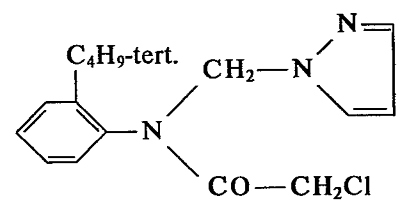

(bekannt aus FR-A 2 379 525)

Beispiel A

Pre-emergence-Test

Lösungsmittel: 5 Gewichtsteile Aceton

Emulgator: 1 Gewichtsteil Alkylarylpolyglycoläther

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0% = keine Wirkung (wie unbehandelte Kontrolle)
100% = totale Vernichtung

Wirkstoffe, Aufwandmengen und Resultate werden ermittelt.

Die erfindungsgemäßen Wirkstoffe gemäß Beispiel 1, 2 und 4 zeigen in diesem Test eine bessere selektive Wirksamkeit als die aus dem Stand der Technik bekannten Verbindungen.

Herstellungsbeispiele

Beispiel 1

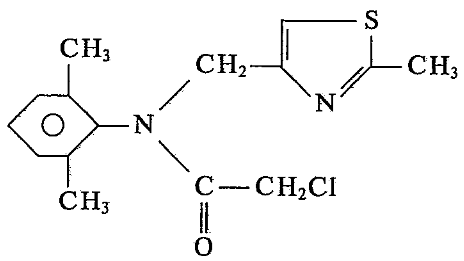

(Verfahren a)

11,6 g (0,05 Mol) 2,6-Dimethyl-N-(2'-methyl-thiazol-4'-yl-methyl)-anilin und 4 g (0,05 Mol) Pyridin werden in 100 ml absolutem Tetrahydrofuran zum Sieden erhitzt und tropfenweise mit 5,7 g (0,05 Mol) Chloracetylchlorid versetzt. Nach 15 Minuten wird die Reaktionslösung im Vakuum eingeengt. Der Rückstand wird mit Wassser versetzt und die organische Phase mit Methylenchlorid extrahiert. Nach Trocknung über Natriumsulfat und Abdestillieren des Lösungsmittels in Vakuum verbleibt ein öliger Rückstand, der durch Anreiben mit Petroläther kristallin wird. Man erhält 12,5 g (81% der Theorie) 2,6-Dimethyl-N-(2'-methyl-thiazol-4'-yl-methyl)-chloracetanilid vom Schmelzpunkt 60 – 62° C.

Herstellung des Ausgangsproduktes

(II-1) 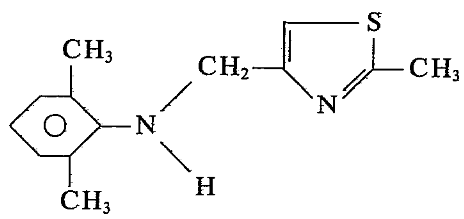

(Verfahren $\alpha$)

60,5 g (0,5 Mol) 2,6-Dimethylanilin und 27,6 g (0,2 Mol) fein gepulvertes Kaliumcarbonat werden in 100 ml Dimethylformamid auf 100° C erhitzt und unter Rühren tropfenweise mit 30 g (0,2 Mol) 4-Chlormethyl-2-methyl-thiazol versetzt. Es wird 5 Stunden bei 100° C gerührt. Danach wird die Reaktionsmischung auf 300 ml Wasser gegossen und mit Methylenchlorid extrahiert. Die organische Phase wird über Natriumsulfat getrocknet und eingeengt. Der Rückstand wird im Vakuum destilliert. Man erhält 28,6 g (63% der Theorie) 2,6-Dimethyl-N-(2'-methyl-thiazol-4'-yl-methyl)-anilin vom Siedepunkt 128 – 130° C/0,05 Torr.

Beispiel 2

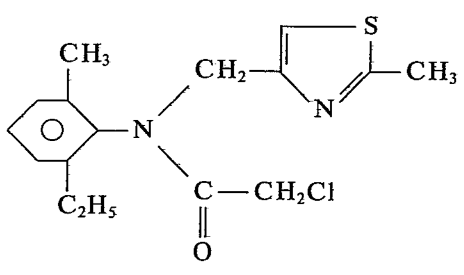

(Verfahren b)

4,8 g (0,022 Mol) 2-Ethyl-6-methyl-chloracetanilid werden in einem Zweiphasengemisch aus 40 ml Toluol und 20 ml 50%iger Natronlauge nach Zugabe von 0,1 g Triethyl-benzyl-ammoniumchlorid gelöst und unter heftigem Rühren tropfenweise mit 4,2 g (0,022 Mol) 4-Brommethyl-2-methyl-thiazol versetzt. Man läßt 3 Stunden bei Raumtemperatur nachrühren. Anschließend wird die Toluolphase abgetrennt,

mehrmals mit Wasser gewaschen und über Natriumsulfat getrocknet. Nach Abdestillieren des Lösungsmittels im Vakuum erhält man 6,3 g (93% der Theorie) 2-Ethyl-6-methyl-N-(2′-methyl-thiazol-4′-yl-methyl)-chloracetanilid als zähes Öl mit einem Brechungsindex von $n_D^{22}$ = 1,573.

Herstellung des Ausgangsproduktes

(IV-1) 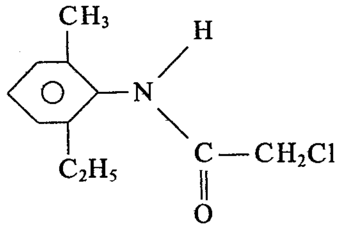

135,2 g (1 Mol) 2-Ethyl-6-methyl-anilin in 1000 ml Toluol werden mit 152 g (1,1 Mol) Kaliumcarbonat versetzt. Dazu werden unter Rühren 113 g (1 Mol) Chloressigsäurechlorid getropft. Nach Abklingen der exothermen Reaktion läßt man 2 Stunden unter Rückfluß nachrühren. Anschließend wird das Reaktionsgemisch filtriert und das Filtrat im Vakuum auf 500 ml eingeengt. Die dabei entstehenden Kristalle werden abgesaugt und mit Petroläther gewaschen. Man erhält 189,6 g (98% der Theorie) 2-Ethyl-6-methyl-chloracet-anilid in Form weißer Kristalle vom Schmelzpunkt 120° C.

In entsprechender Weise werden diejenigen Verbindungen erhalten, die in der Tabelle 1 formelmäßig aufgeführt sind.

Tabelle 1

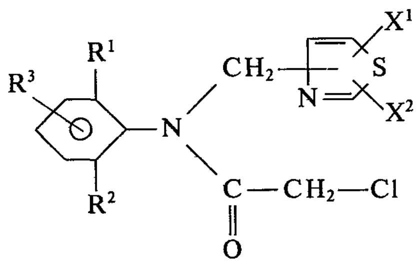

| Beispiel Nr. | $R^1$ | $R^2$ | $R^3$ | 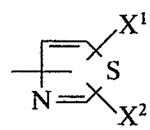 | Fp. (°C) |
|---|---|---|---|---|---|
| 3 | $CH_3$ | $C_2H_5$ | H | 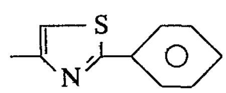 | 87—89 |
| 4 | $C_2H_5$ | $C_2H_5$ | H | 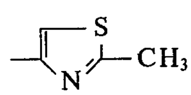 | 62—65 |
| 5 | $C(CH_3)_3$ | H | H | 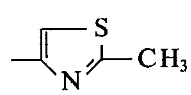 | 85—86 |
| 6 | $CH_3$ | H | 3-$CH_3$ | 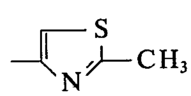 | 72—74 |
| 7 | $C_2H_5$ | $C_2H_5$ | H | 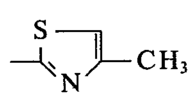 | 95—96 |
| 8 | $C_2H_5$ | $C_2H_5$ | H | 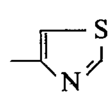 | 71—72 |

Fortsetzung

| Beispiel Nr. | $R^1$ | $R^2$ | $R^3$ | 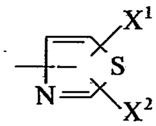 | Fp. (°C) |
|---|---|---|---|---|---|
| 9 | $CH_3$ | $C_2H_5$ | H | 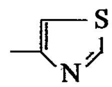 | 42—44 |
| 10 | $CH_3$ | $CH_3$ | H | 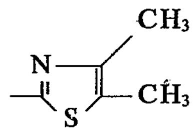 | zähes Öl |
| 11 | $CH_3$ | $CH_3$ | H | 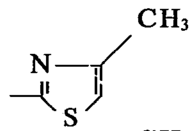 | 63—64 |
| 12 | $CH_3$ | $C_2H_5$ | H | 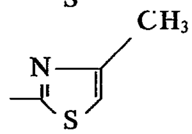 | 85—87 |
| 13 | $CH_3$ | H | 5-$CH_3$ | 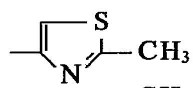 | 76—77 |
| 14 | $CH_3$ | H | 3-$CH_3$ | 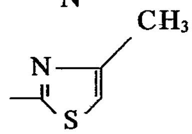 | 59—60 |

**Nach einem oder mehreren der in der Anmeldung beschriebenen Verfahren und entsprechend Beispiel 1 werden die in der nachstehenden Tabelle 2 formelmäßig aufgeführten Ausgangsprodukte der Formel (II) erhalten.**

Tabelle 2

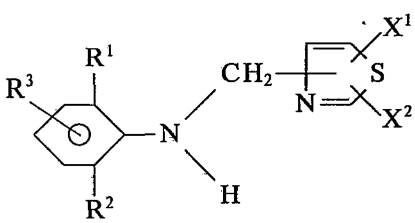

(II)

| Beispiel Nr. | $R^1$ | $R^2$ | $R^3$ | 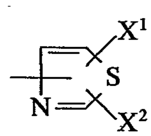 | Kp. |
|---|---|---|---|---|---|
| (II-2) | $CH_3$ | $C_2H_5$ | H | 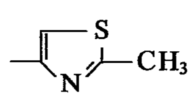 | 149°C/ 0,05 Torr |
| (II-3) | $CH_3$ | $C_2H_5$ | H | 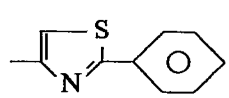 | 188°C/ 0,015 Torr |

Fortsetzung

| Beispiel Nr. | $R^1$ | $R^2$ | $R^3$ | 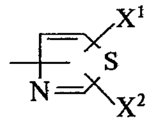 | Kp. |
|---|---|---|---|---|---|
| (II-4) | $C_2H_5$ | $C_2H_5$ | H | 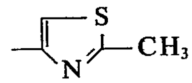 | 140–145°C/ 0,1 Torr |
| (II-5) | $C(CH_3)_3$ | H | H | 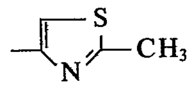 | 153–160°C/ 0,1 Torr |
| (II-6) | $CH_3$ | H | 3-$CH_3$ | 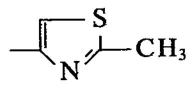 | Fp. 73–74°C |
| (II-7) | $C_2H_5$ | $C_2H_5$ | H | 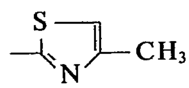 | 150–160°C/ 0,1 Torr |
| (II-8) | $C_2H_5$ | $C_2H_5$ | H | 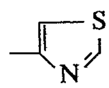 | 150°C/ 0,2 Torr |
| (II-9) | $CH_3$ | $C_2H_5$ | H | 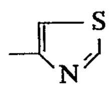 | 128–132°C/ 0,1 Torr |
| (II-10) | $CH_3$ | $CH_3$ | H | 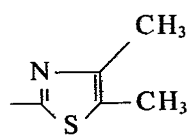 | 140–145°C/ 0,2 Torr |
| (II-11) | $CH_3$ | $CH_3$ | H | 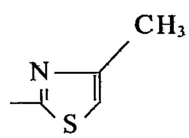 | 142–145°C/ 0,2 Torr |
| (II-12) | $CH_3$ | $C_2H_5$ | H | 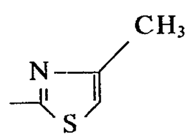 | 144–150°C/ 0,2 Torr |
| (II-13) | $CH_3$ | H | 5-$CH_3$ | 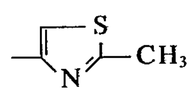 | 131–141°C/ 0,05 mm Hg |
| (II-14) | $CH_3$ | H | 3-$CH_3$ | 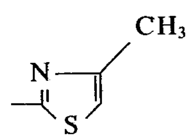 | Fp. 85–86°C |

Nach bekannten Verfahren und entsprechend Beispiel 2 werden die in der nachstehenden Tabelle 3 formelmäßig aufgeführten Ausgangsprodukte der Formel (IV) erhalten.

Tabelle 3

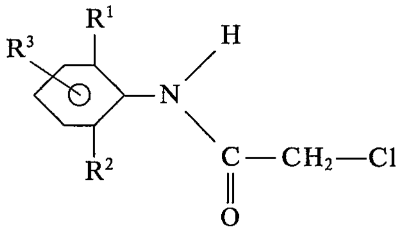

(IV)

| Bsp. Nr. | $R^1$ | $R^2$ | $R^3$ | Schmelzpunkt (°C) |
|---|---|---|---|---|
| (IV-2) | $CH_3$ | $CH_3$ | H | 148 |
| (IV-3) | $C_2H_3$ | $C_2H_5$ | H | 133 |
| (IV-4) | i-$C_3H_7$ | H | H | 79 |
| (IV-5) | tert.-$C_4H_9$ | H | H | 96 |
| (IV-6) | $C_2H_5$ | H | H | 103 |
| (IV-7) | $CH_3$ | H | H | 109 |
| (IV-8) | $CH_3$ | H | 3-$CH_3$ | 135 |
| (IV-9) | $CH_3$ | H | 5-$CH_3$ | 154 |
| (IV-10) | $CH_3$ | $CH_3$ | 4-$CH_3$ | 177 |
| (IV-11) | $C_2H_5$ | $CH_3$ | 4-$CH_3$ | 134 |
| (IV-12) | sek.-$C_4H_9$ | H | H | Öl |
| (IV-13) | H | H | H | 132 |

**Patentansprüche**

1. N-(1,3-Thiazolyl)alkyl-chloracetanilide der Formel

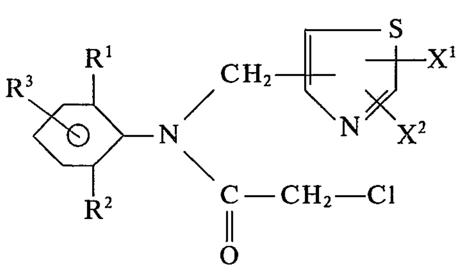

(I)

in welcher

$R^1$ für Alkyl mit 1 bis 4 Kohlenstoffatomen steht,
$R^2$ für Wasserstoff oder Alkyl mit 1 bis 4 Kohlenstoffatomen steht,
$R^3$ für Wasserstoff oder Alkyl mit 1 bis 4 Kohlenstoffatomen steht,
$X^1$ für Wasserstoff, Alkyl mit 1 bis 4 Kohlenstoffatomen oder Phenyl steht,
$X^2$ für Wasserstoff oder Alkyl mit 1 bis 4 Kohlenstoffatomen steht und

der Azolyl-Rest über ein Kohlenstoffatom gebunden ist.

2. Verbindung der Formel

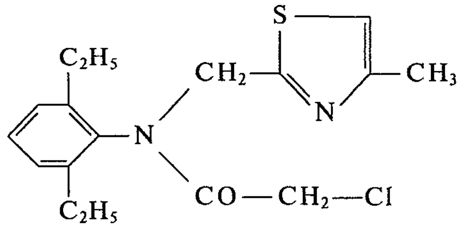

3. Verfahren zur Herstellung von N-(1,3-Thiazolyl)alkylchloracetaniliden der Formel

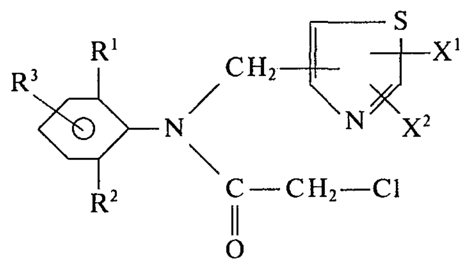

(I)

in welcher

$R^1$ für Alkyl mit 1 bis 4 Kohlenstoffatomen steht,
$R^2$ für Wasserstoff oder Alkyl mit 1 bis 4 Kohlenstoffatomen steht,
$R^3$ für Wasserstoff oder Alkyl mit 1 bis 4 Kohlenstoffatomen steht,
$X^1$ für Wasserstoff, Alkyl mit 1 bis 4 Kohlenstoffatomen oder Phenyl steht,
$X^2$ für Wasserstoff oder Alkyl mit 1 bis 4 Kohlenstoffatomen steht und

der Azolyl-Rest über ein Kohlenstoffatom gebunden ist, dadurch gekennzeichnet, daß man

a) N-(1,3-Thiazolyl)alkyl-aniline der Formel

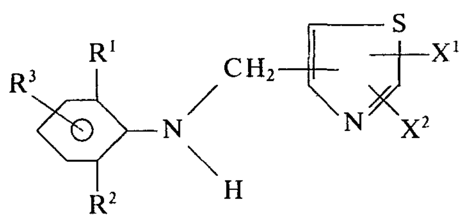

(II)

in welcher

$R^1$, $R^2$, $R^3$, $X^1$ und $X^2$ die oben angegebene Bedeutung haben,

und der Azolyl-Rest über ein Kohlenstoffatom gebunden ist, mit Chloressigsäurechlorid oder -bromid bzw. -anhydrid der Formeln

$$Cl-CH_2-CO-Cl(Br) \qquad (IIIa)$$

bzw.

$$(Cl-CH_2-CO)_2O \qquad (IIIb)$$

in Gegenwart eines Verdünnungsmittels und gegebenenfalls in Gegenwart eines Säurebindemittels umsetzt, oder

b) Chloracetanilide der Formel

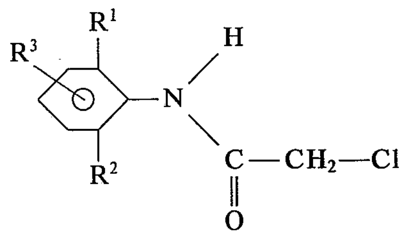

(IV)

in welcher

$R^1$, $R^2$ und $R^3$ die oben angegebene Bedeutung haben,

mit Azolyl-alkyl-Derivaten der Formel

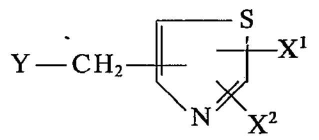

(V)

in welcher

| | |
|---|---|
| $X^1$ und $X^2$ | die oben angegebene Bedeutung haben und |
| Y | für Halogen, den Mesylat- oder Tosylat-Rest steht, und |

der Azolyl-Rest über ein Kohlenstoffatom gebunden ist, in Gegenwart eines Säurebinders und gegebenenfalls in Gegenwart eines organischen Lösungsmittels umsetzt.

4. Herbizide Mittel, gekennzeichnet durch einen Gehalt an mindestens einem N-(1,3-Thiazolyl) alkylchloracetanilid der Formel (I).

5. Herstellung von Verfahren zur herbiziden Mitteln, dadurch gekennzeichnet, daß man N-(1,3-Thiazolyl)alkyl-chloracetanilide der Formel (I) mit Streckmitteln und/oder oberflächenaktiven Mitteln vermischt.

6. Verwendung von N-(1,3-Thiazolyl)alkyl-chloracetaniliden der Formel (I) zur Bekämpfung von Unkräutern.

**Claims**

1. N-(1,3-thiazolyl)alkyl-chloroacetanilides of the formula

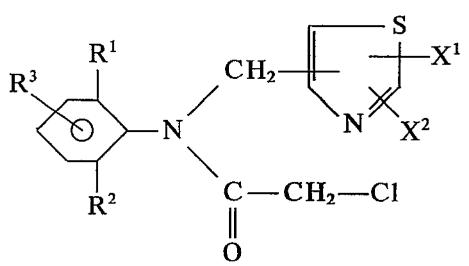

(I)

in which

$R^1$ represents alkyl with 1 to 4 carbon atoms,
$R^2$ represents hydrogen or alkyl with 1 to 4 carbon atoms,
$R^3$ represents hydrogen or alkyl with 1 to 4 carbon atoms,
$X^1$ represents hydrogen, alkyl with 1 to 4 carbon atoms or phenyl,
$X^2$ represents hydrogen or alkyl with 1 to 4 carbon atoms and

the azolyl radical is bonded via a carbon atom.

2. Compound of the formula

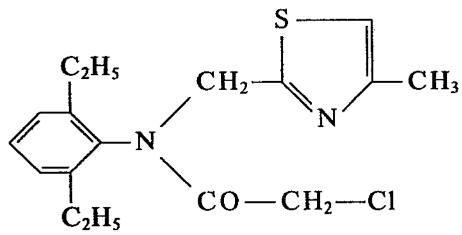

3. Process for the preparation of N-(1,3-thiazolyl)alkyl-chloroacetanilides of the formula

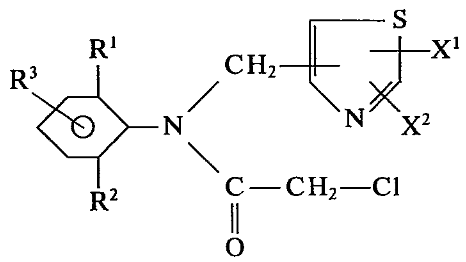 (I)

in which

$R^1$ represents alkyl with 1 to 4 carbon atoms,
$R^2$ represents hydrogen or alkyl with 1 to 4 carbon atoms,
$R^3$ represents hydrogen or alkyl with 1 to 4 carbon atoms,
$X^1$ represents hydrogen, alkyl with 1 to 4 carbon atoms or phenyl,
$X^2$ represents hydrogen or alkyl with 1 to 4 carbon atoms and

the azolyl radical is bonded via a carbon atom, characterised in that

a) N-(1,3-thiazolyl)alkyl-anilines of the formula

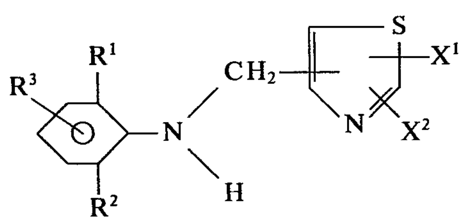 (II)

in which

$R^1$, $R^2$, $R^3$, $X^1$ and $X^2$ have the meaning indicated above

and the azolyl radical is bonded via a carbon atom, are reacted with chloroacetic acid chloride or bromide or anhydride of the formulae

$$Cl-CH_2-CO-Cl(Br) \quad \text{(IIIa)}$$

or

$$(Cl-CH_2-CO)_2O \quad \text{(IIIb)}$$

in the presence of a diluent and if appropriate in the presence of an acid-binding agent, or

b) chloroacetanilides of the formula

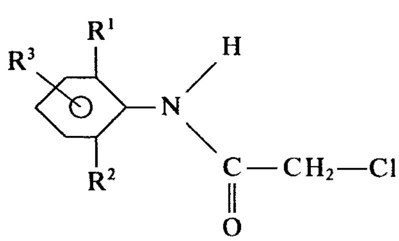 (IV)

in which

$R^1$, $R^2$ and $R^3$ have the meaning indicated above,

are reacted with azolyl-alkyl derivatives of the formula

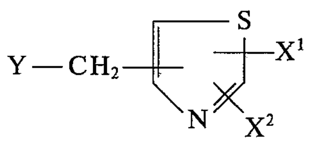

(V)

in which

$X^1$ and $X^2$ have the meaning indicated above and
Y represents halogen or the mesylate or tosylate radical and

the azolyl radical is bonded via a carbon atom, in the presence of an acid-binding agent and if appropriate in the presence of an organic solvent.

4. Herbicidal agents characterised in that they contain at least one N-(1,3-thiazolyl)alkyl-chloroacetanilide of the formula (I).
5. Process for the preparation of herbicidal agents, characterised in that N-(1,3-thiazolyl)alkyl-chloroacetanilides of the formula (I) are mixed with extenders and/or surfaceactive agents.
6. Use of N-(1,3-thiazolyl)alkyl-chloroacetanilides of the formula (I) for combating weeds.

**Revendications**

1. N-(1,3-thiazolyl)alkyl-chloracétanilides de formule:

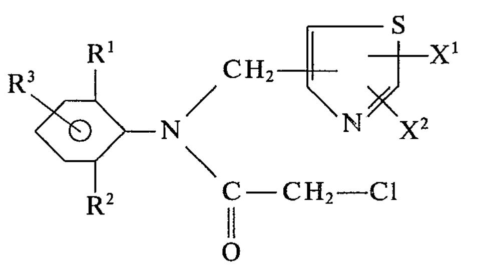

(I)

dans laquelle

$R^1$ représente un groupe alkyle contenant 1 à 4 atomes de carbone,
$R^2$ représente un atome d'hydrogène ou un groupe alkyle contenant 1 à 4 atomes de carbone,
$R^3$ représente un atome d'hydrogène ou un groupe alkyle contenant 1 à 4 atomes de carbone,
$X^1$ représente un atome d'hydrogène, un groupe alkyle contenant 1 à 4 atomes de carbone, ou un groupe phényle,
$X^2$ représente un atome d'hydrogène ou un groupe alkyle contenant 1 à 4 atomes de carbone, et
le radical azolyle est fixé sur un atome de carbone.

2. Composé répondant à la formule:

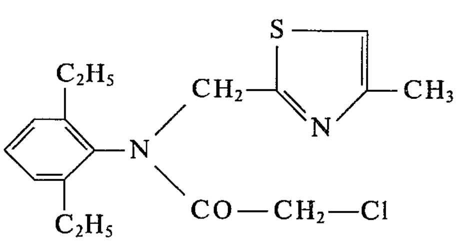

3. Procédé de préparation de N-(1,3-thiazolyl)alkyl-chloracétanilides de formule:

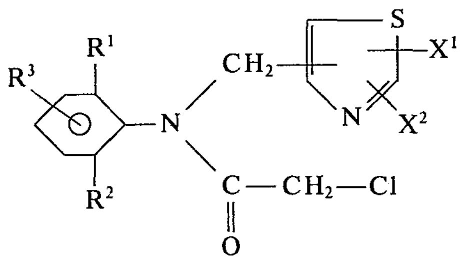

(I)

dans laquelle

$R^1$ représente un groupe alkyle contenant 1 à 4 atomes de carbone,
$R^2$ représente un atome d'hydrogène ou un groupe alkyle contenant 1 à 4 atomes de carbone,
$R^3$ représente un atome d'hydrogène ou un groupe alkyle contenant 1 à 4 atomes de carbone,
$X^1$ représente un atome d'hydrogène, un groupe alkyle contenant 1 à 4 atomes de carbone, ou un groupe phényle,
$X^2$ représente un atome d'hydrogène ou un groupe alkyle contenant 1 à 4 atomes de carbone, et

le radical azolyle est fixé sur un atome de carbone, caractérisé en ce que:

a) on fait réagir des N-(1,3-thiazolyl)alkyl-anilines de formule:

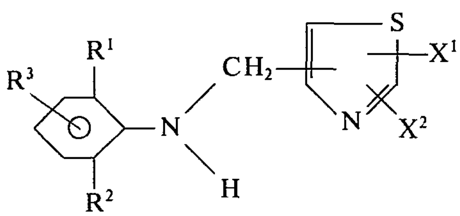

(II)

dans laquelle

$R^1$, $R^2$, $R^3$, $X^1$ et $X^2$ ont les significations indiquées ci-dessus,

tandis que le radical azolyle est fixé sur un atome de carbone,
avec le chlorure, le bromure ou l'anhydride d'acide chloracétique répondant à une des formules:

$$Cl-CH_2-CO-Cl(Br) \quad \text{(IIIa)}$$

et

$$(Cl-CH_2-CO)_2O \quad \text{(IIIb)}$$

en présence d'un diluant et éventuellement en présence d'un agent fixateur d'acide, ou

b) on fait réagir des chloracétanilides de formule:

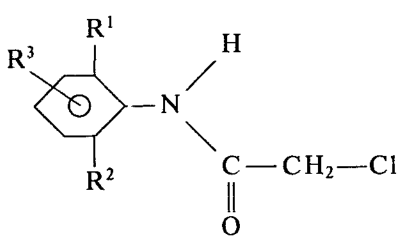

(IV)

dans laquelle

$R^1$, $R^2$ et $R^3$ ont les significations indiquées ci-dessus,

avec des dérivés d'azolyl-alkyle de formule:

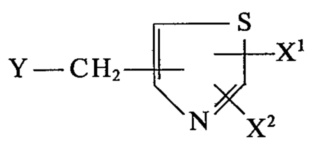

(V)

dans laquelle

| | |
|---|---|
| $X^1$ et $X^2$ | ont les significations indiquées ci-dessus, et |
| Y | représente un atome d'halogène, le radical mésylate ou le radical tosylate, et |

le radical azolyle est fixé sur un atome de carbone, en présence d'un fixateur d'acide et éventuellement en présence d'un solvant organique.

4. Agents herbicides, caractérisés en ce qu'ils contiennent au moins un N-(1,3-thiazolyl)alkyl-chloracétanilide de formule (I).
5. Procédé de préparation d'agents herbicides, caractérisé en ce qu'on mélange des N-(1,3-thiazolyl)alkyl-chloracétanilides de formule (I) avec des diluants et/ou des agents tensio-actifs.
6. Utilisation de N-(1,3-thiazolyl)alkyl-chloracétanilides de formule (I) pour combattre les plantes adventices.